# EUROPEAN PATENT SPECIFICATION

(11) **EP 4 090 252 B1**
(45) Date of publication and mention of the grant of the patent: **20.05.2026**
(21) Application number: 21707434.3
(22) Date of filing: 14.01.2021
(51) Int. Cl.: A61B 17/00, A61B 17/11, A61F 2/06

(54) **MEDICAL DEVICES FOR SHUNTS, OCCLUDERS, FENESTRATIONS AND RELATED SYSTEMS**
MEDIZINISCHE VORRICHTUNGEN FÜR SHUNTS, OKKLUDER, FENESTRATIONEN UND VERWANDTE SYSTEME
DISPOSITIFS MÉDICAUX POUR SHUNTS, OCCLUDEURS, FENESTRATIONS ET SYSTÈMES ASSOCIÉS

(30) Priority: 17.01.2020 US 202062962540 P
(43) Date of publication of application: 23.11.2022
(73) Proprietor: W. L. Gore & Associates, Inc., Newark, DE 19711 (US)
(72) Inventor: COLE, Daniel S., Newark, Delaware 19711 (US); MCDANIEL, Tom R., Newark, Delaware 19711 (US); SHAW, Edward E., Newark, Delaware 19711 (US); SMITH, Benjamin A., Newark, Delaware 19711 (US); VEITH, McKenzie, Newark, Delaware 19711 (US); HAARSTAD, Phil, Newark, Delaware 19711 (US); JAEGER, Jacob, Newark, Delaware 19711 (US)
(74) Representative: HGF
(86) International application number: PCT/US2021/013421
(87) International publication number: WO 2021/146412

(56) References cited:
- WO-A2-2009/137755
- US-A1- 2014 012 368
- US-A1- 2014 222 040
- US-A1- 2015 142 049
- US-A1- 2015 313 599
- US-A1- 2019 231 510

## Description

### CROSS-REFERENCE TO RELATED APPLICATION

This application claims the benefit of Provisional Application No. 62/962,540, filed January 17, 2020.

### FIELD

The present disclosure relates generally to implantable medical devices, and more specifically to implantable medical devices for shunting and/or occluding bodily fluids or structures and related systems.

### BACKGROUND

Heart failure and diseases of the heart affect millions of people worldwide. Heart failure includes failure of either the left side of the heart, the right side of the heart, or both. Diseases of the heart that can lead to heart failure include hypertension, pulmonary arterial hypertension, and congenital defects of the heart. The constantly evolving nature of heart failure represents a significant challenge for the treatment methods. Therefore, there is a need for new and adaptable methods and devices for treating heart failure. US2014012368 describes devices and methods to enhance implantation, retrievability, or repositionability. The devices include pivotable sections providing the ability to maintain the device's engagement with a delivery system during implantation where the delivery system approaches an opening of the septum at an angle. The devices also include configurations that allow for improved retrieval into a delivery system in the event that a malfunction or a problem with the patients physiology is detected.

### SUMMARY

The invention is defined according to the appended claims which relate to an implantable medical device and a delivery system for deploying an implantable medical device. The invention is defined in independent claim 1. Certain optional features of the invention are defined in the dependent claims. According to one example ("Example 1"), an implantable medical device includes a first frame component configured to conform to an anatomy of a patient; a second frame component configured to conform to an anatomy of a patient wherein the first frame component and the second frame component are discrete and separate from one another, and at least one of the first frame component and the second frame component includes inner apices and outer apices, and one or more eyelets arranged with at least one of the outer apices and the inner apices; and a conduit portion arranged between the first frame component and the second frame component, the conduit portion including a membrane connecting the first frame component and the second frame component.

According to one example ("Example 2"), further to the device of Example 1, at least a portion of the conduit portion is radially unsupported by the first and second frame components within the conduit portion.

According to one example ("Example 3"), further to the device of Example 2, the first and second frame components are configured to facilitate deployment of the conduit portion and maintaining a lumen through the conduit portion.

According to one example ("Example 4"), further to the device of any one of Examples 1-3, the conduit portion is free of frame components.

According to one example ("Example 5"), further to the device of any one of Examples 1-4, the first frame component includes a first set of elongate elements and the second frame component includes a second set of elongate elements, and the first set of elongate elements and the second set of elongate elements are non-contiguous with one another.

According to one example ("Example 6"), further to the device of Example 5, the first set of elongate elements include a first plurality of support struts and wherein the second set of elongate elements include a second plurality of support struts, the first and second plurality of support struts forming a support structure within each of the elongate elements.

According to one example ("Example 7"), further to the device of Example 6, the first set of elongate elements form a plurality of first lobes.

According to one example ("Example 8"), further to the device of Example 6, the first set of elongate elements form a star shape.

According to one example ("Example 9"), further to the device of any one of Examples 1-8, at least one of the first frame component and the second frame component includes a star shape having the inner apices and the outer apices.

According to one example ("Example 10"), further to the device of Example 9, the at least one of the first frame component and the second frame component includes an exterior side and an interior side, and the one or more eyelets are arranged on either side of at least one of the inner apices and the outer apices.

According to one example ("Example 11"), further to the device of Example 10, each of the inner apices includes one of the eyelets arranged on the exterior side of the at least one of the first frame component and the second frame component and each of the outer apices includes one of the eyelets arranged on the interior side of the at least one of the first frame component and the second frame component.

According to one example ("Example 12"), further to the device of Example 9, the eyelets arranged with the inner apices are open to the exterior side of the at least one of the first frame component and the second frame component and the eyelets arranged with the outer apices are open to the interior side of the at least one of the first frame component and the second frame component.

According to one example ("Example 13"), further to the device of Example 9, at least one of the first frame component and the second frame component includes a curved star shape.

According to one example ("Example 14"), further to the device of Example 13, the eyelets are open to the interior side of the at least one of the first frame component and the second frame component.

According to one example ("Example 15"), further to the device of Example 9, at least one of the first frame component and the second frame component includes linear portions arranged between the eyelets and the outer apices.

According to one example ("Example 16"), further to the device of Example 9, wherein the eyelets are oval shaped.

According to one example ("Example 17"), further to the device of Example 9, the eyelets are arranged with the outer apices and are open to the interior side of the at least one of the first frame component and the second frame component.

According to one example ("Example 18"), further to the device of Example 1, wherein at least one of the first frame component and the second frame component includes a star shape having the inner apices and the outer apices and elongate elements having variable widths or one or more curvatures between the outer apices and the inner apices.

According to one example ("Example 19"), further to the device of Example 1, wherein at least one of the first frame component and the second frame component includes a star shape having the inner apices and the outer apices and elongate elements connected by interconnecting struts near the inner apices.

According to one example ("Example 20"), a delivery system for deploying the device of any one of Examples 1-19, the delivery system include one or more engagement elements, one or more release lines arranged through the engagement elements and configured to pass through the one or more eyelets to couple the at least one of the first frame component and the second frame component to the delivery system.

According to one example ("Example 21"), further to the system of Example 21, wherein the one or more engagement elements are configured to collapse the at least one of the first frame component and the second frame component toward a catheter.

According to one example ("Example 22"), further to the system of any one of Examples 20-21, the release lines are configured to withdraw from the one or more eyelets to release the frame component from the delivery system.

According to another example ("Example 23"), a method, which is not part of the invention, for regulating blood pressure between a left and right atrium of a heart includes delivering the implantable medical device to a desired treatment location within a body of a patient, the implantable medical device includes a conduit portion configured to span a septum of the heart and configured to allow fluid flow therethrough; a frame component including a first set of elongate elements arranged on a first side of the conduit portion and a second set of elongate elements arranged on a second side of the conduit portion with the first set of elongate elements and the second set of elongate elements being non-contiguous with one another and at least one of the first frame component and the second frame component includes inner apices and outer apices, and one or more eyelets arranged with at least one of the outer apices and the inner apices; positioning the device such that the conduit portion spans a septum between the left and right atrium of the heart; and deploying the first frame component and the second frame component such that the conduit portion opens a desired amount to provide a fluid flow path between the left and right atrium.

According to another example ("Example 24"), further to the method of Example 23, the method also includes adjusting tension on the device to adjust a diameter of the conduit portion and a fluid flow velocity therethrough.

According to another example ("Example 25"), an implantable medical device includes a first frame component configured to conform to an anatomy of a patient; a second frame component configured to conform to an anatomy of a patient wherein the first frame component and the second frame, and at least one of the first frame component and the second frame component includes inner apices and outer apices; and wherein at least one of the first frame component and the second frame component includes a star shape having the inner apices and the outer apices and the at least one of the first frame component and the second frame component includes an exterior side and an interior side, and one or more eyelets are arranged on either side of at least one of the inner apices and the outer apices.

According to another example ("Example 26"), further to the device of Example 25, each of the inner apices includes one of the eyelets arranged on the exterior side of the at least one of the first frame component and the second frame component and each of the outer apices includes one of the eyelets arranged on the interior side of the at least one of the first frame component and the second frame component.

According to another example ("Example 27"), further to the device of Example 25, at least one of the first frame component and the second frame component includes an exterior side and an interior side, and one or more eyelets are arranged with the inner apices are open to the exterior side of the at least one of the first frame component and the second frame component and the eyelets arranged with the outer apices are open to the interior side of the at least one of the first frame component and the second frame component.

According to another example ("Example 28"), further to the device of Example 25, the star shape is a curved star shape.

According to another example ("Example 29"), further to the device of Example 28, the eyelets are open to the interior side of the at least one of the first frame component and the second frame component.

According to another example ("Example 30"), further to the device of Example 25, the one or more eyelets are arranged on either side of at least one of the inner apices and the outer apices and at least one of the first frame component and the second frame component includes linear portions arranged between the eyelets and the outer apices.

According to another example ("Example 31"), further to the device of any one of Examples 25-30, the eyelets are oval shaped.

According to another example ("Example 32"), further to the device of Example 25, the one or more eyelets arranged with the outer apices and are open to the interior side of the at least one of the first frame component and the second frame component.

According to another example ("Example 33"), further to the device of Example 25, at least one of the first frame component and the second frame component includes the star shape having elongate elements with variable widths or one or more curvatures between the outer apices and the inner apices.

According to another example ("Example 34"), further to the device of Example 25, at least one of the first frame component and the second frame component includes the star shape having elongate elements with elongate elements connected by interconnecting struts near the inner apices.

According to another example ("Example 35"), a delivery system for deploying an implantable medical device for regulating blood pressure between a left and right atrium of a heart, the implantable medical device including a first frame component configured to conform to an anatomy of a patient, a second frame component configured to conform to an anatomy of a patient wherein the first frame component and the second frame, and at least one of the first frame component and the second frame component includes inner apices and outer apices, and wherein at least one of the first frame component and the second frame component includes a star shape having the inner apices and the outer apices and the at least one of the first frame component and the second frame component includes an exterior side and an interior side, and one or more eyelets are arranged on either side of at least one of the inner apices and the outer apices, the system comprising: one or more engagement elements; and one or more release lines arranged through the engagement elements and configured to pass through the one or more eyelets to couple the at least one of the first frame component and the second frame component to the delivery system.

According to another example ("Example 36"), further to the system of Example 35, the one or more engagement elements are configured to collapse the at least one of the first frame component and the second frame component toward a catheter.

According to another example ("Example 37"), further to the system of any one of Examples 35-36, the release lines are configured to withdraw from the one or more eyelets to release the frame component from the delivery system.

According to another example ("Example 38"), further to the system of Example 35, each of the inner apices includes one of the eyelets arranged on the exterior side of the at least one of the first frame component and the second frame component and each of the outer apices includes one of the eyelets arranged on the interior side of the at least one of the first frame component and the second frame component.

According to another example ("Example 39"), further to the system of Example 35, at least one of the first frame component and the second frame component includes an exterior side and an interior side, and one or more eyelets are arranged with the inner apices are open to the exterior side of the at least one of the first frame component and the second frame component and the eyelets arranged with the outer apices are open to the interior side of the at least one of the first frame component and the second frame component.

According to another example ("Example 40"), a method, which is not part of the invention, for regulating blood pressure between a left and right atrium of a heart includes delivering the implantable medical device to a desired treatment location within a body of a patient, the implantable medical device including: a conduit portion configured to span a septum of the heart and configured to allow fluid flow therethrough; a frame component including a first set of elongate elements arranged on a first side of the conduit portion and a second set of elongate elements arranged on a second side of the conduit portion with the first set of elongate elements and the second set of elongate elements being non-contiguous with one another, and at least one of the first frame component and the second frame component includes inner apices and outer apices, and at least one of the first frame component and the second frame component includes a star shape having the inner apices and the outer apices and the at least one of the first frame component and the second frame component includes an exterior side and an interior side, and one or more eyelets are arranged on either side of at least one of the inner apices and the outer apices; positioning the device such that the conduit portion spans a septum between the left and right atrium of the heart; and deploying the first frame component and the second frame component such that the conduit portion opens a desired amount to provide a fluid flow path between the left and right atrium.

According to another example ("Example 41"), further to the method of Example 40, the method also includes adjusting tension on the device to adjust a diameter of the conduit portion and a fluid flow velocity therethrough.

According to another example ("Example 42"), further to the method of Example 40, positioning the device includes engaging one or more eyelets with one or more engagement elements; and arranging one or more release lines through the engagement elements and configured to pass through the one or more eyelets to couple the at least one of the first frame component and the second frame component a delivery system.

According to another example ("Example 43"), further to the method of Example 42, the one or more engagement elements are configured to collapse the at least one of the first frame component and the second frame component toward a catheter.

According to another example ("Example 44"), further to the method of Example 42, the release lines are configured to withdraw from the one or more eyelets to release the frame component from the delivery system.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is an example implantable medical device for regulating blood pressure.
FIG. 2 is an example implantable medical device for regulating blood pressure.
FIG. 3A is a perspective view of another example implantable medical device for regulating blood pressure.
FIG. 3B is a side view of the implantable medical device for regulating blood pressure, shown in FIG. 3A.
FIG. 4 shows an example frame component that may be used in an implantable medical device for regulating blood pressure in accordance with .
FIG. 5 shows another example frame component that may be used in an implantable medical device for regulating blood pressure in accordance with
FIG. 6 shows an example frame component that may be used in an implantable medical device for regulating blood pressure in accordance with the appended claims.
FIG. 7 shows an example frame component that may be used in an implantable medical device for regulating blood pressure in accordance with the appended claims.
FIG. 8 shows an example frame component that may be used in an implantable medical device for regulating blood pressure.
FIG. 9 shows an example frame component that may be used in an implantable medical device for regulating blood pressure.
FIG. 10A shows a top view of an example frame component that may be used in an implantable medical device for regulating blood pressure in accordance with the appended claims.
FIG. 10B shows a side view of the frame component, shown in FIG. 10A, in accordance with the appended claims.
FIG. 11 shows an example frame component that may be used in an implantable medical device for regulating blood pressure.
FIG. 12 shows an example frame component that may be used in an implantable medical device for regulating blood pressure in accordance with the appended claims.
FIG. 13A shows a top view of an example frame component that may be used in an implantable medical device for regulating blood pressure.
FIG. 13B shows a side view of the frame component, shown in FIG. 13A.
FIG. 14A shows a top view of an example frame component that may be used in an implantable medical device for regulating blood pressure in accordance with the appended claims
FIG. 14B shows a side view of the frame component, shown in FIG. 14A, in accordance with the appended claims.
FIG. 14C shows a perspective view of the frame component, shown in FIGS. 14A-B, in accordance with the appended claims.
FIG. 15A shows a top view of an example frame component that may be used in an implantable medical device for regulating blood pressure in accordance with the appended claims.
FIG. 15B shows a side view of the frame component, shown in FIG. 15A, in accordance with the appended claims.
FIG. 15C shows a perspective view of the frame component, shown in FIGS. 15A-B, in accordance with the appended claims.
FIG. 16A shows a cut pattern of an example frame component that may be used in an implantable medical device for regulating blood pressure.
FIG. 16B shows a side view of the frame component, shown in FIG. 16A
FIG. 17 shows an example deployment system and frame component, in accordance with the appended claims.
FIG. 18 shows an end portion of an example deployment system and frame component, in accordance with the appended claims.
FIG. 19 shows example graspers for recapturing a frame component.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward to implantable medical devices such as device for shunting and/or occluding bodily fluids or structures. In certain instances, the various aspects of the present disclosure relate to methods and devices for treating heart failure by reducing elevated blood pressure in a heart chamber by creating a pressure relief shunt. Additionally, some embodiments relate to devices for customizing, adjusting or manipulating the flow of blood through the shunt in order to enhance the therapeutic effect of the pressure relief shunt.

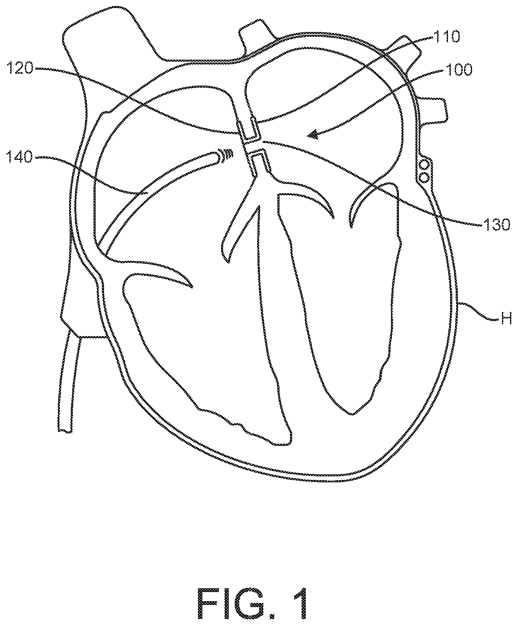

FIG. 1 is an example implantable medical device for regulating blood pressure. The implantable medical device 100 is shown implanted within a heart H of a patient. The device 100 is shown arranged between the patient's left atrium and right atrium. In certain instances, the device 100 may be used to regulate blood flow within the heart H, for example, between the left and right atriums LA, RA. As shown, the device 100 generally includes a first frame component 110 arranged on a first side of a septum (e.g., within the right atrium RA), a second frame component 120 arranged on a second side of the septum (e.g., within the left atrium LA), and a conduit portion 130 extending through the septum. A needle may be used to create an opening in the septum.

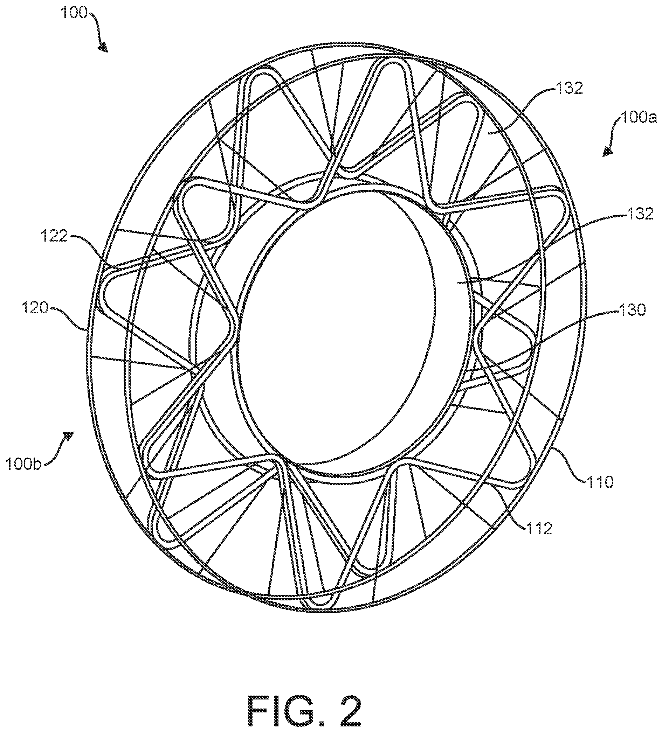

A sheath 140 and constraining and/or release lines (not shown) may be used to facilitate deployment of the device 100. For example, a first side of the device 100 that includes the first frame component 110 may be released after the sheath 140 is advanced through the septum and to the RA, and the second frame component 120 that includes the second frame component 120 may be released on the LA side of the septum. A conduit portion 130 (*e*.*g*., shown in FIG. 2) is arranged within the opening. The frame components 110, 120 and the conduit portion 130 may be compressed within the sheath 140 during delivery of the device 100 to the desired treatment area within the patient and subsequently expanded during deployment of the device 100.

FIG. 2 is an example implantable medical device for regulating blood pressure. As shown, the device 100 includes the first frame component 110 and the second frame component 120. The first frame component 110 may be configured to conform to the patient's anatomy (i.e., the first side of the septum, for example). The second frame component 120 may be configured to conform to the patient's anatomy (i.e., the second side of the septum).

In certain instances, the first frame component 110 includes a first set of elongate elements 112, and the second frame component 120 includes a second set of elongate elements 122. The frame components 110, 120, including and for example the elongate elements 112, 122, may be discrete and separate from one another. For example, the first frame component 110 forms a first side 100a of the device 100 and the second frame component 120 forms a second side 100b of the device 100. The first frame component 110 being discrete and separate from the second frame component 120 does not enter into the second side 100b of the device and the second frame component 120 being discrete and separate from the first frame component 110 does not enter into the first side 100a of the device.

In certain instances, the first and second frame components 110, 120 are non-contiguous with one another. The first and second frame components 110, 120 being non-contiguous with one another allows the first and second frame components 110, 120 to be distinct and separate from one another. In addition, the first and second frame components 110, 120 are free to move, in response to movement of the patient's anatomy, separate from one another. In this manner, forces acting on one of the first and second frame components 110, 120 are maintained within the other of the first and second frame components 110, 120. The forces acting on one of the first and second frame components 110, 120 may be isolated to the frame component to which the force is acted on.

As shown, the conduit portion 130 is arranged between the first frame component and the second frame component. At least a portion of the conduit portion 130 is generally radially or circumferentially unsupported by the first and second frame components 110, 120 within the conduit portion 130. As shown in FIG. 2, the conduit portion 130 transitions to the first side 100a and the second side 100b at approximately a 90 degree angle (other angles are contemplated). Bounds of the conduit portion 130 may be considered to be a location at which the conduit portion 130 transitions to the first side 100a and the second side 100b. The first and second frame components 110, 120 extend laterally relative to the conduit portion 130. In addition, the first and second frame components 110, 120 may support the conduit portion 130 without substantially entering the bounds of the conduit portion 130. In certain instances, the first and second frame components 110, 120 support the conduit portion 130 laterally from outside of bounds the conduit portion 130. Thus, the first and second frame components 110, 120 may maintain a lumen through the conduit portion 130 and facilitate deployment of the conduit portion 130 by laterally forcing the conduit portion 130 open.

In certain instances, the first and second frame components 110, 120 may impart tension to the conduit portion 130 to deploy and maintain the conduit portion 130 with a lumen therethrough. The conduit portion 130 may be deployed within the septum between tissue surfaces through an opening (*e*.*g*., needle stick across the septum) that has a diameter smaller than a fully deployed diameter of the conduit portion 130. Tension in the conduit portion 130 imparted by expansion of the first and second frame components 110, 120 may also expand the septum between tissue surfaces to a desired shunt size.

In certain instances, the conduit portion 130 may be substantially free of frame components. For example, because the first and second frame components 110, 120 are non-contiguous with one another, as described above, and are arranged external to the bounds of the conduit portion 130. The conduit portion 130 may include, for example, a membrane 132, such as an expanded polytetrafluoroethylene (ePTFE) membrane, connecting the first frame component 110 and the second frame component 120. The membrane 132 generally separates the first frame component 110 and the second frame component 120 by a suitable distance compatible with the patient's body. For example, the membrane 132 can separate the first frame component 110 and the second frame component 120 by a gap of from 0 to 15 mm depending on the desired treatment location within the patient's body. In addition, the conduit portion may be formed of only the membrane 132. The conduit portion 130, which is configured to be deployed within the septum between tissue surfaces, is free of the first frame component 110 and the second frame component 120. The conduit portion 130 may include a smooth interior that facilitates blood flow therethrough without ridges from a stent element interrupting or disrupting flow. Thus, the conduit portion 130 may lessen the opportunity for thrombosis.

In addition to the membrane 132 forming the conduit portion 130, the membrane 132 may also cover at least a portion of the first frame component 110, at least a portion of the second frame component 120, or at least a portion of the first frame component 110 and the second frame component 120. In certain instances, the membrane 132 arranged on at least a portion of the first frame component 110 and/or the second frame component 120 is a separate membrane film (*e*.*g*., a first membrane film arranged on first frame component 110 and a second membrane film arranged on the second frame component 120). In these instances, the membrane film or films may be coupled to the membrane 132 in the conduit portion 130. The membrane 132 may be elastic to allow for expansion of the conduit portion 130 and to allow for movement of portions of the first frame component 110 and/or the second frame component 120 (*e*.*g*., movement of the first set of elongate elements 112 and/or the second set of elongate elements 122).

The membrane 132 may span gaps between the first set of elongate elements 112 and/or the second set of elongate elements 122. The membrane 132, in certain instances, is arranged on at least a tissue engaging side of the first frame component 110 and a tissue engaging side the second frame component 120. In these instances, the membrane 132 is configured to lessens frame erosion potential of the first frame component 110 and/or the second frame component 120. The membrane 132 and the arrangement of the first set of elongate elements 112 and/or the second set of elongate elements 122 may conform to the tissue surfaces surrounding the septum. The first set of elongate elements 112 and/or the second set of elongate elements 122 may lay flat against the tissue surfaces.

In certain instances, each of the first set of elongate elements 112 may be attached to one another via the membrane 132 to form the first frame component 110. In certain instances, the first frame component 110 may form a substantially flat or 2-dimensional, disc-like shape, as shown. Additionally, or alternatively, the second set of elongate elements 122 may also be attached to one another via the membrane material 132 to form the second frame component 120. The second frame component 120 may also form a substantially flat or 2-dimensional, disc-like shape such that the first and second frame components 110, 120 are substantially parallel to one another when the device 100 is in a deployed configuration.

In certain instances, the membrane 132 may be configured to promote tissue ingrowth over at least a portion of the membrane 132, or at least a portion of the membrane 132. In certain instances, the membrane 132 is configured to promote tissue ingrowth to cover at least a portion of the first and/or second frame components 110, 120, which may further promote compatibility and stability of the device 100 within the patient's body. The membrane 132 within the conduit portion 130 may be configured to not allow tissue ingrowth leading to increased patency. In certain instances, the membrane 132 is configured to promote endothelization without obstructive ingrowth within the conduit portion 130. The membrane 132 may promote endothelization without obstructive overgrowth of tissue into the conduit portion 130.

In certain instances, the device 100 may be capable of delivering a drug to the desired treatment location within the patient's body. For example, the device 100 may be capable of eluting a drug configured to modulate tissue response. In certain instances, the device 100 may be coated with a therapeutic coating, drug eluting material or other therapeutic material or a hydrophilic coating. In one specific example, the device 100 can be coated with heparin to facilitate thromboresistance and patency of the device 100. Alternatively, or additionally, the device 100 may include paclitaxel (to modulate tissue/cellular response).

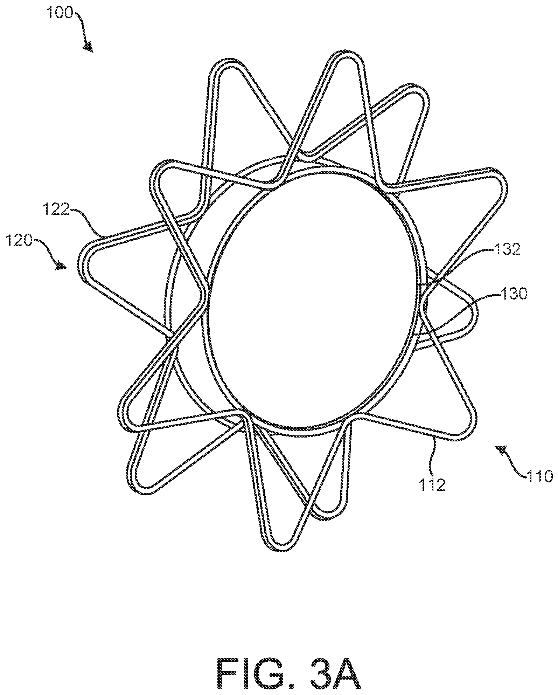

FIG. 3A is a perspective view of another example implantable medical device 100 for regulating blood pressure. As shown, each of the first set of elongate elements 112 may be discrete and separate from adjacent elongate elements. In other terms, the membrane 132 does not connect each of the first set of elongate elements 112 together. In this way, each of the first set of elongate elements 112 may move independently from one another and individually conform to the topography of the first side of the septum, thus providing a highly conformable first frame component 110. Each of the second set of elongate elements 122 may also be discrete and separate from adjacent elongate elements. For example, each of the second set of elongate elements 122 may move independently from one another and individually conform to the second side of the septum, much like the first set of elongate elements 112 conforms to the first side of the septum. Thus, both the first and second frame components 110, 120 are highly conformable and may conform independently of one another based on the patient's anatomy.

In certain instances, one of the first or second set of elongate elements 112, 122 of the first and second frame components 110, 120 may be attached to one another via the membrane 132 while the other set of elongate elements are unattached (e.g., they are discrete and separate from adjacent elongate elements). In other instances, only some of the first or second set of elongate elements 112, 122 may be attached to one another, while other elongate elements of the first and second set of elongate elements 112, 122 are not attached. Thus, the device 100 can be highly customizable to the patient depending on the desired treatment location within the patient, and size and/or shape of the defect, among other factors.

The device 100 is generally deployable or expandable from a delivery configuration to the deployed configuration. In some instances, the first set of elongate elements 112 and the second set of elongate elements 122 may nest within one another when the device is in the delivery configuration. This allows the device 100 to compress to a smaller size, for example, for delivery of the device 100 to a wider variety of treatment locations (e.g., through small, narrow, or convoluted passageways).

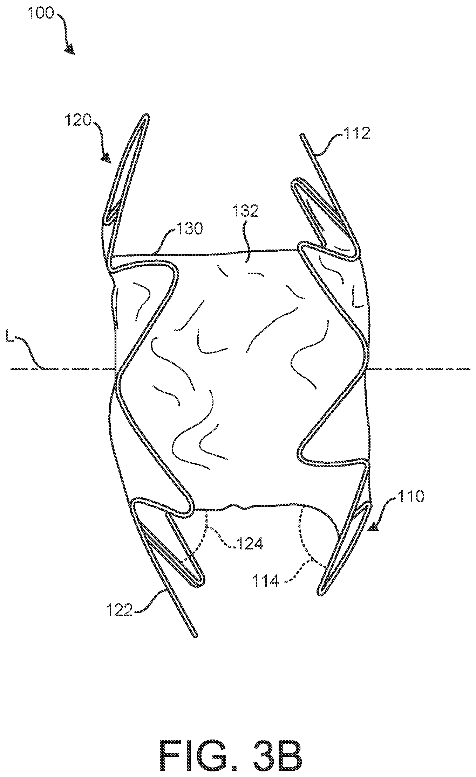

FIG. 3B is a side view of the implantable medical device for regulating blood pressure, shown in FIG. 3A. FIG. 3B shows the device 100 in the deployed configuration. As shown, the first frame component 110 including the first set of elongate elements 112 and the second frame component 120 including the second set of elongate elements 122 are positioned radially outward with respect to a longitudinal axis L of the conduit portion 130 when the device 100 is in the deployed configuration. For example, the first and second frame components 110, 120 are positioned at first and second angles 114, 124, respectively. The first and second angles 114, 124 may form approximately a 90° angle with respect to the longitudinal axis L when the device is in the deployed configuration. This allows the first and second frame components 110, 120 to be positioned parallel with and adjacent to the first and second sides of the septum. In certain instances, the first and second frame components 110, 120 may be positioned at any angle relative to the longitudinal axis L (for example, from about 0° to greater than 90° with respect to the longitudinal axis L) that allows for contact with the tissue surface of the first and second sides of the septum.

In certain instances, the first and second elongate elements 112, 122 are configured to separate from one another when the device 100 is in the deployed configuration. As shown in FIG. 3B, each of the first set of elongate elements 112 are discrete and separate from one another when the device 100 is in the deployed configuration such that each of the first set of elongate elements 112 may move independently from adjacent elongate elements. Each of the second set of elongate elements 122 may also be discrete and separate from one another when the device 100 is in the deployed configuration such that each of the second set of elongate elements 122 move independently from adjacent elongate elements.

The first and second frame components 110, 120 may maintain a lumen through the conduit portion 130 and facilitate deployment of the conduit portion 130 by laterally forcing the conduit portion 130 open. In addition, the lumen may be free or without the first and second frame components 110, 120. In this manner, the conduit portion 130 may facilitate re-crossing of the septum for addition procedures (e.g., left atrial appendage occluder implantation). In addition, the first and second frame components 110, 120 may be differently configured. For example, one of the first and second frame components 110, 120 may be flared while the other of the first and second frame components 110, 120 is flat. In other instances, both the first and second frame components 110, 120 may be flared. In addition, one of the first and second frame components 110, 120 may be convex while the other of the first and second frame components 110, 120 is flat or concave or both the first and second frame components 110, 120 may be convex. Further, one of the first and second frame components 110, 120 may be concave while the other of the first and second frame components 110, 120 is flat or convex or both the first and second frame components 110, 120 may be concave. In addition, the first and second frame components 110, 120 may be different sizes.

The first and second frame components 110, 120 may include a sensor integrated into the respective frame component, for example, for continuous monitoring of various hemodynamic parameters such as pressure, among other parameters, within the patient's body. For example, an antenna or inductor may be wrapped around the perimeter of one of the first and second frame components 110, 120 and the sensor may be attached to the inductor. The sensor may be configured to, for example, sense physiologic properties, such as temperature, electrical signals of the heart, blood chemistry, blood pH level, hemodynamics, biomarkers, sound, pressure, and electrolytes that may be important in diagnosing, monitoring, and/or treating heart disease, heart failure, and/or other cardiovascular disease states

In certain instances, the conduit portion 130 may be sizeable after delivery. The membrane 132 may be selectively adjustable by a balloon applied within the conduit portion 130 to distend the membrane 132. The device 100 can be any size suitable to fit the anatomy of the patient. In certain instances, a diameter of the conduit portion is from 3 to 12 mm. For example, the diameter of the conduit portion may be from 4 to 10 mm, or from 5 to 8 mm depending on the anatomy of the patient and/or the desired treatment location. The first and second frame components 110, 120 generally have a larger diameter than that of the conduit portion 130, for example, so that the frame components may anchor the conduit portion 130 of the device 100 within the septum.

The device 100 can be any shape suitable to fit the anatomy of the patient. For example, the first and second frame portions 110, 120 may be any of a variety of suitable shapes for anchoring the device 100 within the patient's body. For example, the first and second frame portions 110, 120 may be substantially circular, ovular, diamond-shaped, star-shaped, flower-shaped, or any other suitable shape as desired. In certain instances, for example, at least one of the first and second set of elongate elements 112, 122 form a star shape. In certain instances, both the first and second set of elongate elements 112, 122 form a star shape.

In certain instances, the first set of elongate elements 112 forms a plurality of first lobes 116 and the second set of elongate elements 122 forms a plurality of second lobes 126. Each of the plurality of first and second lobes 116, 126 may include, for example, from 3 to 12 lobes, from 4 to 10 lobes, or from 6 to 8 lobes as desired. In certain instances, the plurality of first lobes 116 may have more lobes than the plurality of second lobes 126, while in other instances, the plurality of first lobes 116 may have the same number of lobes or less lobes than the plurality of second lobes 126.

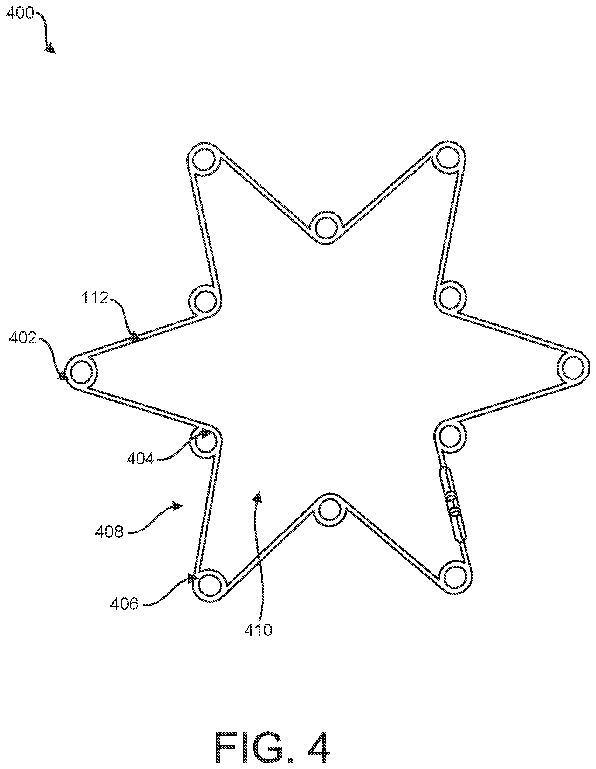

FIG. 4 shows an example frame component 400 that may be used in an implantable medical device for regulating blood pressure. The frame component 400 may be used in place of one or both of the frame components 110, 120 shown and discussed above with reference to FIGS. 1-3. For example, the frame component 400 may take place of the first and/or second frame components 110, 120 maintain a lumen through a conduit portion 130. The frame component 400 may include elongate elements 112 that form the frame component 400. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 400 and form the conduit portion 130.

The membrane may span gaps between the elongate elements 112, and, in certain instances, may be arranged on at least a tissue engaging side of the frame component 400. In these instances, the membrane is configured to lessens frame erosion potential of the frame component 400. The elongate elements 112 may conform to the tissue surfaces surrounding the septum. In addition, the elongate elements 112 may lay flat against the tissue surfaces.

In certain instances, the elongate elements 112 form a star shape as is shown in FIG. 4. The star-shaped frame component 400 includes outer apices 402 and inner apices 404. In certain instances, the frame component 400 may include one or more eyelets 406 arranged with at least one of the outer apices 402 and the inner apices 404. The frame component 400 also includes an exterior side 408 and an interior side 410. In certain instances, the one or more eyelets 406 may be arranged on either side of the inner apices 404 and/or the outer apices 402. In certain instances, each of the inner apices 404 includes one of the eyelets 406 arranged on the exterior side 408 of the frame 400. In addition, each of the outer apices 402 may include one of the eyelets 406 arranged on the interior side 410 of the frame 400 in certain instances. In other instances, one of the eyelets 406 may be arranged on the exterior side 408 of the frame 400 without eyelets arranged on the interior side 410 of the frame 400. In yet other instances, one of the eyelets 406 may be arranged on the interior side 410 of the frame 400 without eyelets 406 arranged on the exterior side 408 of the frame 400. The eyelets 406 may be configured to interface with a delivery system to facilitate deployment of the frame component 400.

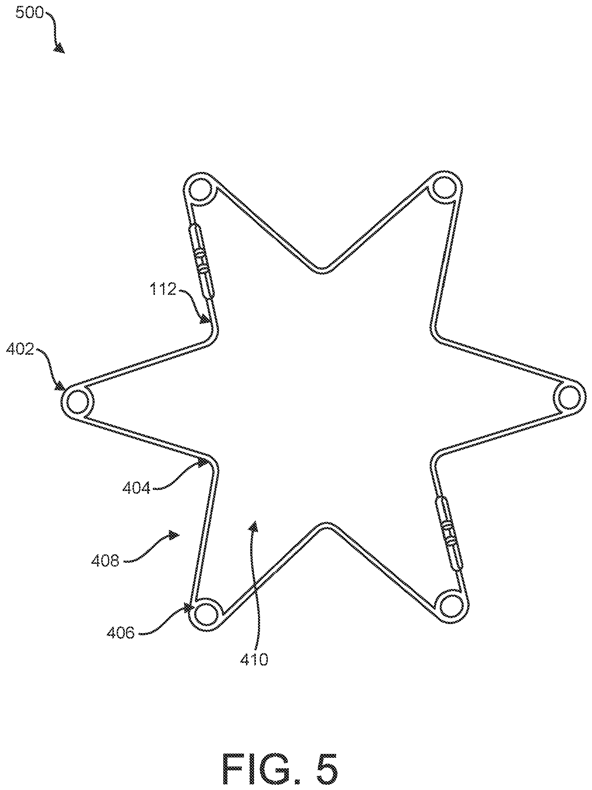

FIG. 5 shows another example frame component that may be used in an implantable medical device for regulating blood pressure. The frame component 500 may be used in place of one or both of the frame components 110, 120 shown and discussed above with reference to FIGS. 1-3. For example, the frame component 500 may take place of the first and/or second frame components 110, 120 maintain a lumen through a conduit portion 130. The frame component 500 may include elongate elements 112 that form the frame component 500. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 500 and form the conduit portion 130.

The membrane may span gaps between the elongate elements 112, and, in certain instances, may be arranged on at least a tissue engaging side of the frame component 500. In these instances, the membrane is configured to lessens frame erosion potential of the frame component 500. The elongate elements 112 may conform to the tissue surfaces surrounding the septum. In addition, the elongate elements 112 may lay flat against the tissue surfaces.

In certain instances, the elongate elements 112 form a star shape as is shown in FIG. 5. The frame component 500 includes outer apices 402 and inner apices 404. In certain instances, the frame component 500 may include one or more eyelets 406 arranged with at least one of the outer apices 402 and the inner apices 404. The frame component 500 also includes an exterior side 408 and an interior side 410. In certain instances, the one or more eyelets 406 may be arranged on either side of the inner apices 404 and/or the outer apices 402. As shown, eyelets 406 are arranged on the interior side 410 of each of the outer apices 402. The eyelets 406 may be configured to interface with a delivery system to facilitate deployment of the frame component 500.

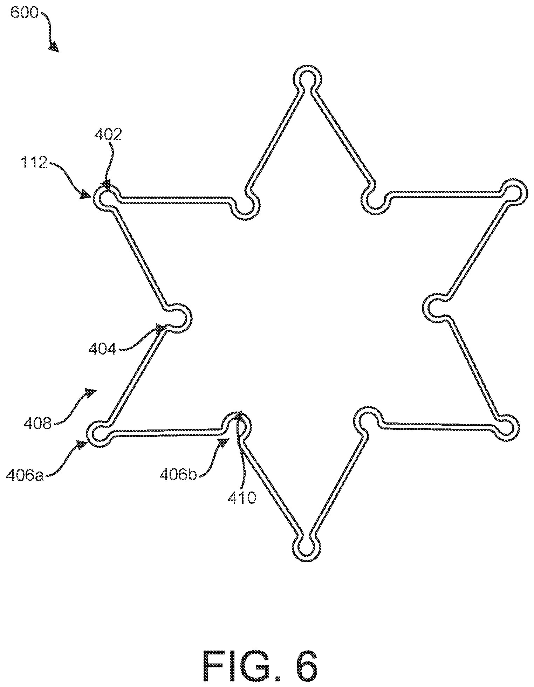

FIG. 6 shows an example frame component 600 that may be used in an implantable medical device for regulating blood pressure in accordance with the claimed implantable medical device. The frame component 600 may be used in place of one or both of the frame components 110, 120 shown and discussed above with reference to FIGS. 1-3. For example, the frame component 600 may take place of the first and/or second frame components 110, 120 maintain a lumen through a conduit portion 130. The frame component 600 may include elongate elements 112 that form the frame component 600. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 600 and form the conduit portion 130.

The membrane may span gaps between the elongate elements 112, and, in certain instances, may be arranged on at least a tissue engaging side of the frame component 600. In these instances, the membrane is configured to lessens frame erosion potential of the frame component 600. The elongate elements 112 may conform to the tissue surfaces surrounding the septum. In addition, the elongate elements 112 may lay flat against the tissue surfaces.

In certain instances, the elongate elements 112 form a star shape as is shown in FIG. 6. The frame component 600 includes outer apices 402 and inner apices 404. In certain instances, the frame component 600 may include one or more eyelets 406a, 406b arranged with at least one of the outer apices 402 and the inner apices 404. The frame component 600 also includes an exterior side 408 and an interior side 410. In certain instances, the one or more eyelets 406a, 406b may be arranged on either side of the inner apices 404 and/or the outer apices 402. In certain instances, the eyelets 406a, 406b may be arranged with both the inner apices 404 and the outer apices 402. In addition, the eyelets 406b arranged with the inner apices 404 are open to the exterior side 408 of the frame component 600 and the eyelets 406a arranged with the outer apices 402 are open to the interior side 410 of the frame component 600. The eyelets 406 may be configured to interface with a delivery system to facilitate deployment of the frame component 500.

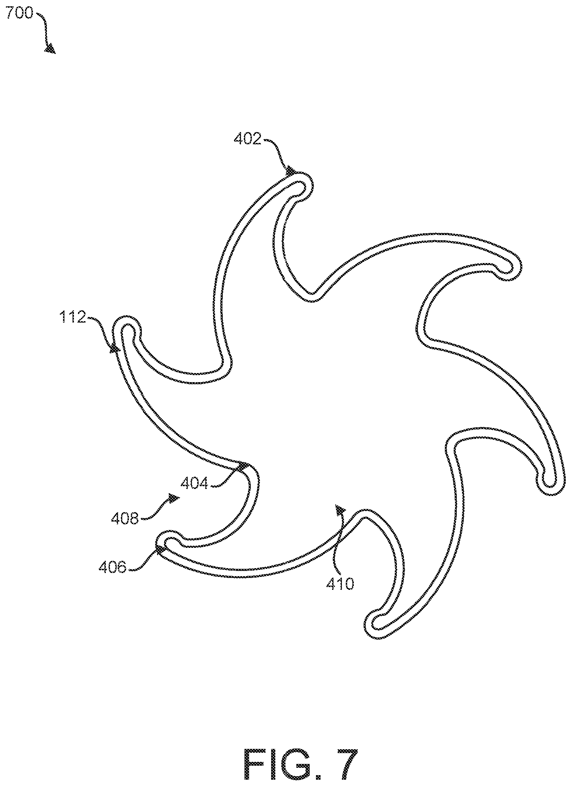

FIG. 7 shows an example frame component that may be used in an implantable medical device for regulating blood pressure in accordance with the claimed implantable medical device. The frame component 700 may be used in place of one or both of the frame components 110, 120 shown and discussed above with reference to FIGS. 1-3. For example, the frame component 700 may take place of the first and/or second frame components 110, 120 maintain a lumen through a conduit portion 130. The frame component 700 may include elongate elements 112 that form the frame component 700. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect star-shaped frame components 700 and form the conduit portion 130.

The membrane may span gaps between the elongate elements 112, and, in certain instances, may be arranged on at least a tissue engaging side of the frame component 700. In these instances, the membrane is configured to lessens frame erosion potential of the frame component 700. The elongate elements 112 may conform to the tissue surfaces surrounding the septum. In addition, the elongate elements 112 may lay flat against the tissue surfaces.

In certain instances, the elongate elements 112 form a curved star shape as is shown in FIG. 7. The star-shaped frame component 700 includes elongate elements 112 having curvatures between apices 402. The frame component 700 includes outer apices 402 and inner apices 404. In certain instances, the frame component 700 may include one or more eyelets 406 arranged with at least one of the outer apices 402 and the inner apices 404. The frame component 400 also includes an exterior side 408 and an interior side 410. In certain instances, the one or more eyelets 406 may be arranged on either side of the inner apices 404 and/or the outer apices 402. In certain instances, the eyelets 406 may be arranged with both the inner apices 404 and the outer apices 402. In addition and as shown, the eyelets 406 may be arranged with the outer apices 402 and may be open to the interior side 410 of the frame component 700. The eyelets 406 may be configured to interface with a delivery system to facilitate deployment of the frame component 700.

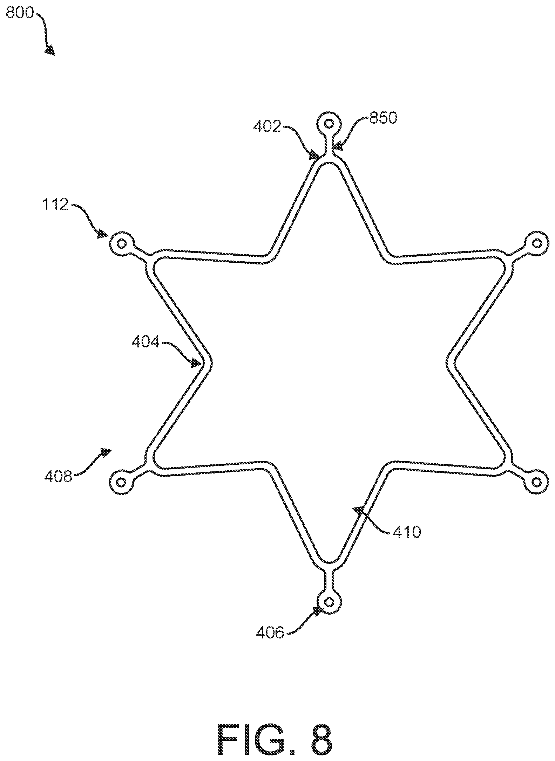

FIG. 8 shows an example frame component 800 that may be used in an implantable medical device for regulating blood pressure. The frame component 800 may be used in place of one or both of the frame components 110, 120 shown and discussed above with reference to FIGS. 1-3. For example, the frame component 800 may take place of the first and/or second frame components 110, 120 maintain a lumen through a conduit portion 130. The frame component 800 may include elongate elements 112 that form the frame component 800. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 800 and form the conduit portion 130.

The membrane may span gaps between the elongate elements 112, and, in certain instances, may be arranged on at least a tissue engaging side of the frame component 800. In these instances, the membrane is configured to lessens frame erosion potential of the frame component 800. The elongate elements 112 may conform to the tissue surfaces surrounding the septum. In addition, the elongate elements 112 may lay flat against the tissue surfaces.

In certain instances, the elongate elements 112 form a star shape as is shown in FIG. 8. The frame component 800 includes outer apices 402 and inner apices 404. In certain instances, the frame component 800 may include one or more eyelets 406 arranged with at least one of the outer apices 402 and the inner apices 404. The frame component 800 also includes an exterior side 408 and an interior side 410. In certain instances, the one or more eyelets 406 may be arranged on either side of the inner apices 404 and/or the outer apices 402. As shown, the eyelets 406 extend outwardly relative to the frame component 800 from the outer apices 402. The frame component 800 includes linear portions 850 arranged between the eyelets 406 and the outer apices 402. The eyelets 406 may be configured to interface with a delivery system to facilitate deployment of the frame component 800.

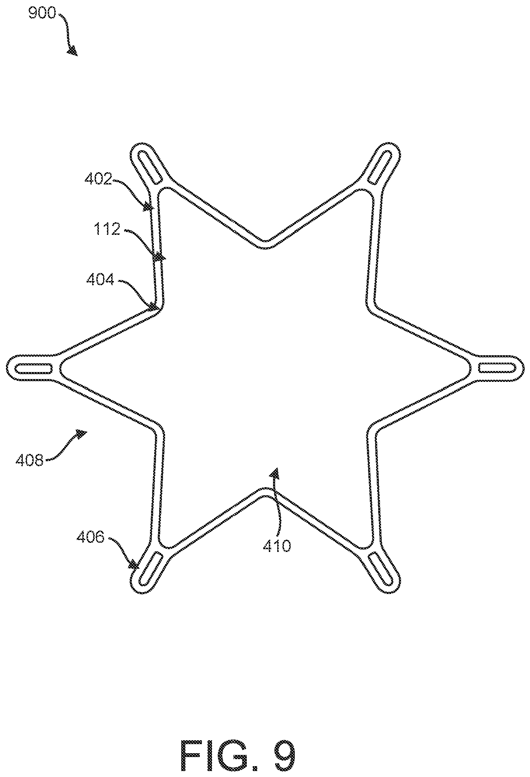

FIG. 9 shows an example frame component 900 that may be used in an implantable medical device for regulating blood pressure. The frame component 900 may be used in place of one or both of the frame components 110, 120 shown and discussed above with reference to FIGS. 1-3. For example, the frame component 900 may take place of the first and/or second frame components 110, 120 maintain a lumen through a conduit portion 130. The frame component 900 may include elongate elements 112 that form the frame component 900. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 900 and form the conduit portion 130.

The membrane may span gaps between the elongate elements 112, and, in certain instances, may be arranged on at least a tissue engaging side of the frame component 900. In these instances, the membrane is configured to lessens frame erosion potential of the frame component 900. The elongate elements 112 may conform to the tissue surfaces surrounding the septum. In addition, the elongate elements 112 may lay flat against the tissue surfaces.

In certain instances, the elongate elements 112 form a star shape as is shown in FIG. 9. The frame component 900 includes outer apices 402 and inner apices 404. In certain instances, the frame component 900 may include one or more eyelets 406 arranged with at least one of the outer apices 402 and the inner apices 404. The frame component 900 also includes an exterior side 408 and an interior side 410. In certain instances, the one or more eyelets 406 may be arranged on either side of the inner apices 404 and/or the outer apices 402. As shown, the eyelets 406 extend outwardly relative to the frame component 900 from the outer apices 402. In addition, the eyelets 406 may be oval shaped. The eyelets 406 may be configured to interface with a delivery system to facilitate deployment of the frame component 900.

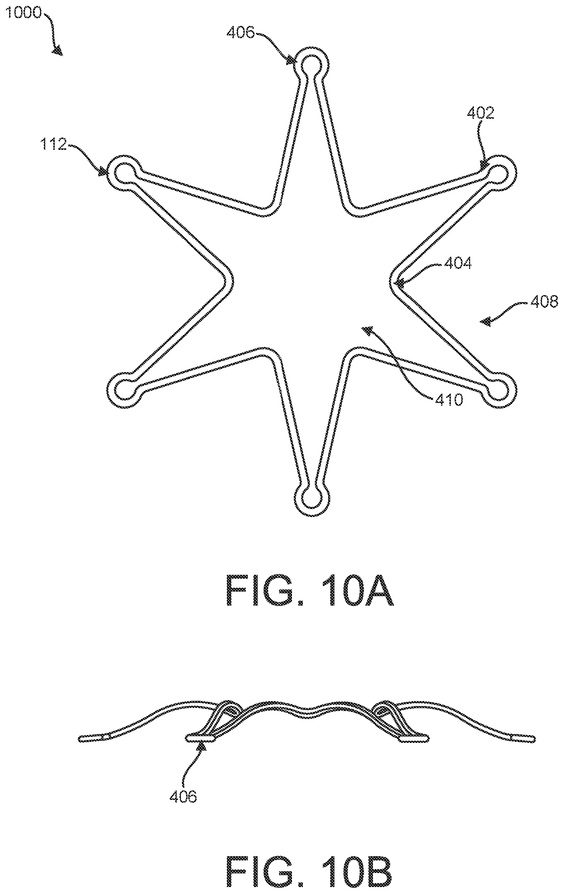

FIG. 10A shows a top view of an example frame component 100 that may be used in an implantable medical device for regulating blood pressure in accordance with the claimed implantable medical device. The frame component 1000 may be used in place of one or both of the frame components 110, 120 shown and discussed above with reference to FIGS. 1-3. For example, the frame component 1000 may take place of the first and/or second frame components 110, 120 maintain a lumen through a conduit portion 130. The frame component 1000 may include elongate elements 112 that form the frame component 1000. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 1000 and form the conduit portion 130.

The membrane may span gaps between the elongate elements 112, and, in certain instances, may be arranged on at least a tissue engaging side of the frame component 1000. In these instances, the membrane is configured to lessens frame erosion potential of the frame component 1000. The elongate elements 112 may conform to the tissue surfaces surrounding the septum. In addition, the elongate elements 112 may lay flat against the tissue surfaces.

In certain instances, the elongate elements 112 form a star shape as is shown in FIG. 10. The frame component 1000 includes outer apices 402 and inner apices 404. In certain instances, the frame component 1000 may include one or more eyelets 406 arranged with at least one of the outer apices 402 and the inner apices 404. The frame component 1000 also includes an exterior side 408 and an interior side 410. In certain instances, the one or more eyelets 406 may be arranged on either side of the inner apices 404 and/or the outer apices 402. As shown, the eyelets 406 are arranged with the outer apices 402 and are open to the interior side 410 of the frame component 1000. FIG. 10B shows a side view of the frame component 1000, shown in FIG. 10A, in accordance with an embodiment. in certain instances, the frame component 1000 includes a variable slope. The elongate elements 112 may include a curvature (e.g., increasing a height of the frame component 1000) as the elongate elements 112 extend inwardly from the outer apices 402.

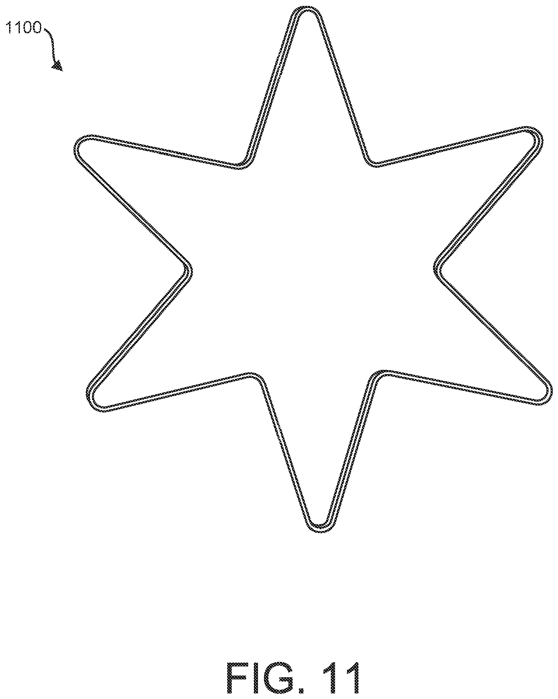

FIG. 11 shows an example frame component 1100 that may be used in an implantable medical device for regulating blood pressure. As compared to other frame components, discussed herein, the frame component 1100 does not include eyelets. Any of the frame component shapes or other features (e.g., as shown in FIGS. 2-10 and 12-16) may also not include eyelets.

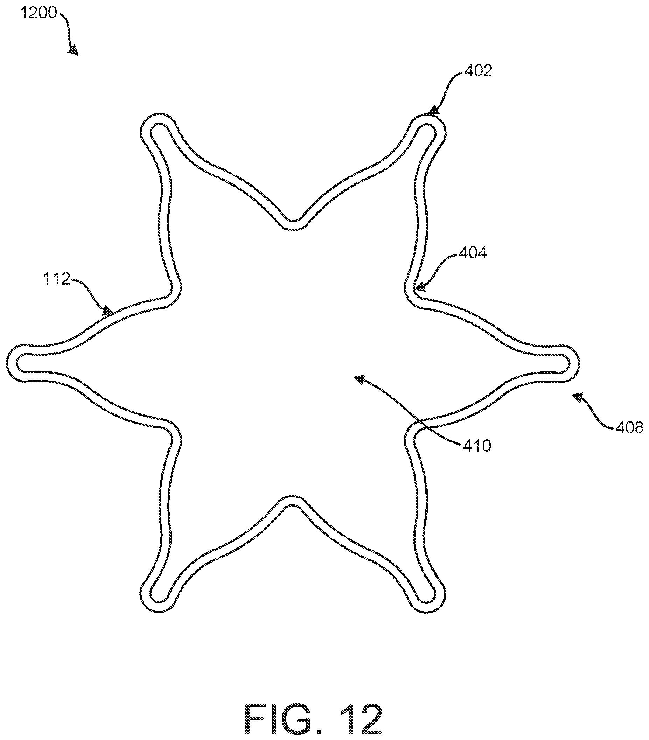

FIG. 12 shows an example frame component 1200 that may be used in an implantable medical device for regulating blood pressure in accordance with the claimed implantable medical device. The frame component 1200 may be used in place of one or both of the frame components 110, 120 shown and discussed above with reference to FIGS. 1-3. For example, the frame component 1200 may take place of the first and/or second frame components 110, 120 maintain a lumen through a conduit portion 130. The frame component 1200 may include elongate elements 112 that form the frame component 1200. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 1200 and form the conduit portion 130.

The membrane may span gaps between the elongate elements 112, and, in certain instances, may be arranged on at least a tissue engaging side of the frame component 1200. In these instances, the membrane is configured to lessens frame erosion potential of the frame component 1200. The elongate elements 112 may conform to the tissue surfaces surrounding the septum. In addition, the elongate elements 112 may lay flat against the tissue surfaces.

In certain instances, the elongate elements 112 form a star shape as is shown in FIG. 12. The frame component 1200 includes outer apices 402 and inner apices 404. The frame component 1200 also includes an exterior side 408 and an interior side 410. In certain instances, the elongate elements 112 may include variable widths or one or more curvatures between the outer apices 402 and the inner apices 404 as shown. In addition, the elongate elements 112 have a curved pattern near the outer apices 402 such that the elongate elements 112 may taper the frame component 1200 inwardly at the outer apices 402. In this instance, the outer apices 420 may be configured as eyelets.

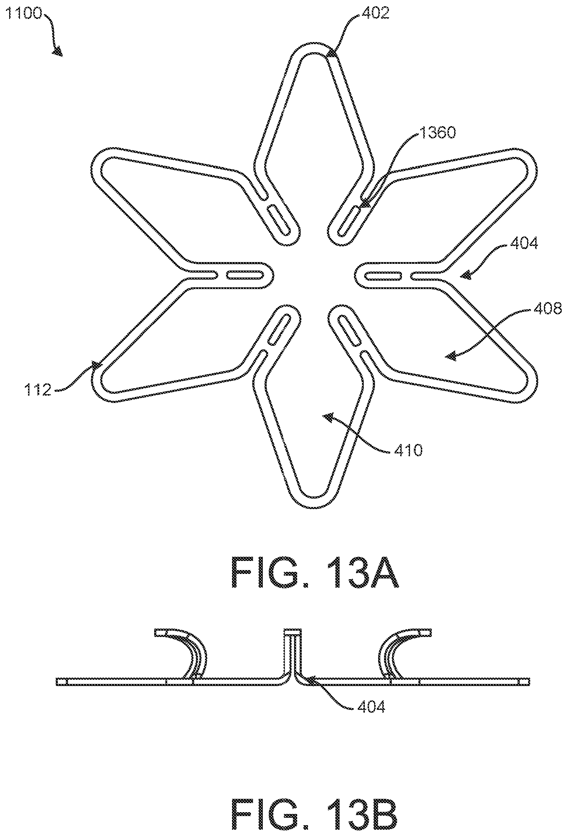

FIG. 13A shows a top view of an example frame component 1300 that may be used in an implantable medical device for regulating blood pressure. The frame component 1300 may be used in place of one or both of the frame components 110, 120 shown and discussed above with reference to FIGS. 1-3. For example, the frame component 1300 may take place of the first and/or second frame components 110, 120 maintain a lumen through a conduit portion 130. The frame component 1300 may include elongate elements 112 that form the frame component 1300. The elongate elements 112 may formed of a wire, cut-tube, or cut-sheet, for example. In certain instances, the elongate elements 112 may be attached to one another via a membrane (not shown). In addition and as discussed in detail above, the membrane such as an expanded polytetrafluoroethylene (ePTFE) membrane, may connect frame components 1300 and form the conduit portion 130.

The membrane may span gaps between the elongate elements 112, and, in certain instances, may be arranged on at least a tissue engaging side of the frame component 1300. In these instances, the membrane is configured to lessens frame erosion potential of the frame component 1300. The elongate elements 112 may conform to the tissue surfaces surrounding the septum. In addition, the elongate elements 112 may lay flat against the tissue surfaces.

In certain instances, the elongate elements 112 form a star shape as is shown in FIG. 13. The frame component 1300 includes outer apices 402 and inner apices 404. The frame component 1300 also includes an exterior side 408 and an interior side 410. In certain instances, the elongate elements 112 are connected by interconnecting struts 1360 near the inner apices 404. In addition and as shown in FIG. 13B the inner apices 404 and may curve the frame component 1300 to change a height of the frame component 1300.

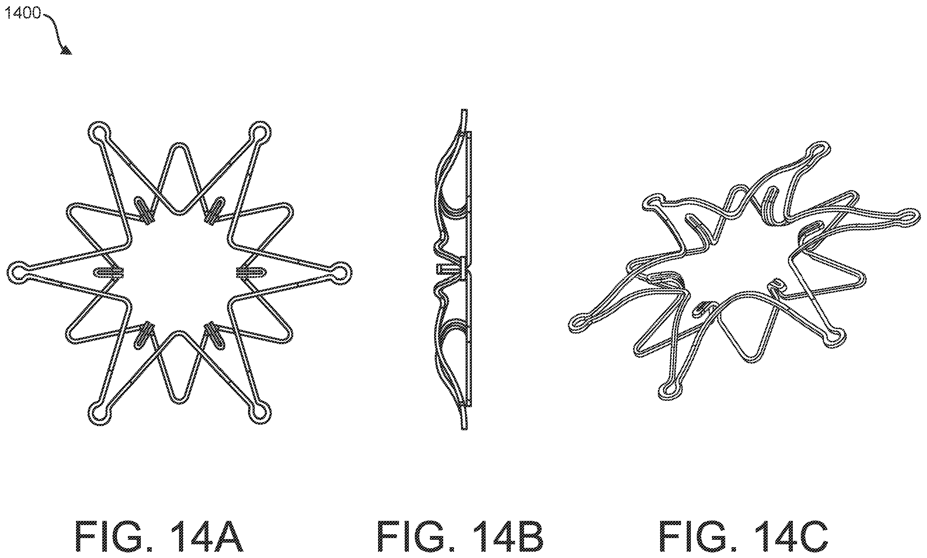

FIGS. 14A shows a top view of an example frame component 400 that may be used in an implantable medical device for regulating blood pressure in accordance with the claimed implantable medical device. FIG. 14B shows a side view of the frame component 1400, shown in FIG. 14A. FIG. 14C shows a perspective view of the frame component 1400, shown in FIGS. 14A-B, in accordance with the claimed implantable medical device. The frame component 1400 may be a combination of other frame components or aspects of frame components shown and discussed above. For example, the frame component 1400 may include aspects of frame component 1300 (*e*.*g*., interconnecting struts 1360 near the inner apices 404) and frame component 600 (*e*.*g*., eyelets 406a arranged with the outer apices 402 are open to the interior side 410 of the frame component 600).

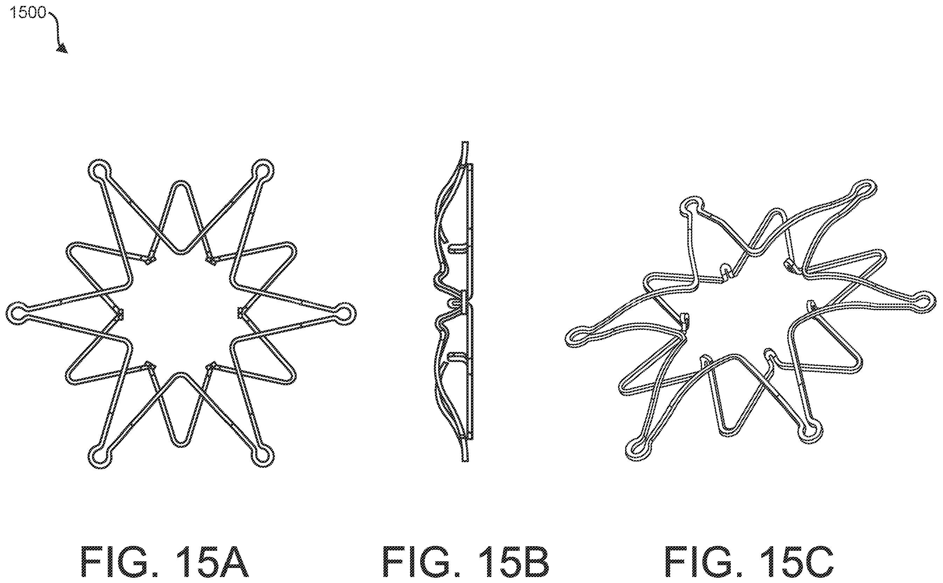

FIG. 15A shows a top view of an example frame component 1500 that may be used in an implantable medical device for regulating blood pressure in accordance with the claimed implantable medical device. FIG. 15B shows a side view of the frame component 1500, shown in FIG. 15A. FIG. 15C shows a perspective view of the frame component 1500, shown in FIGS. 15A-B in accordance with the claimed implantable medical device. The frame component 1500 may be a combination of other frame components or aspects of frame components shown and discussed above. For example, the frame component 1500 may include aspects of frame component 1300 (*e.g*., interconnecting struts 1360 near the inner apices 404) and frame component 600 (*e*.*g*., eyelets 406a arranged with the outer apices 402 are open to the interior side 410 of the frame component 600).

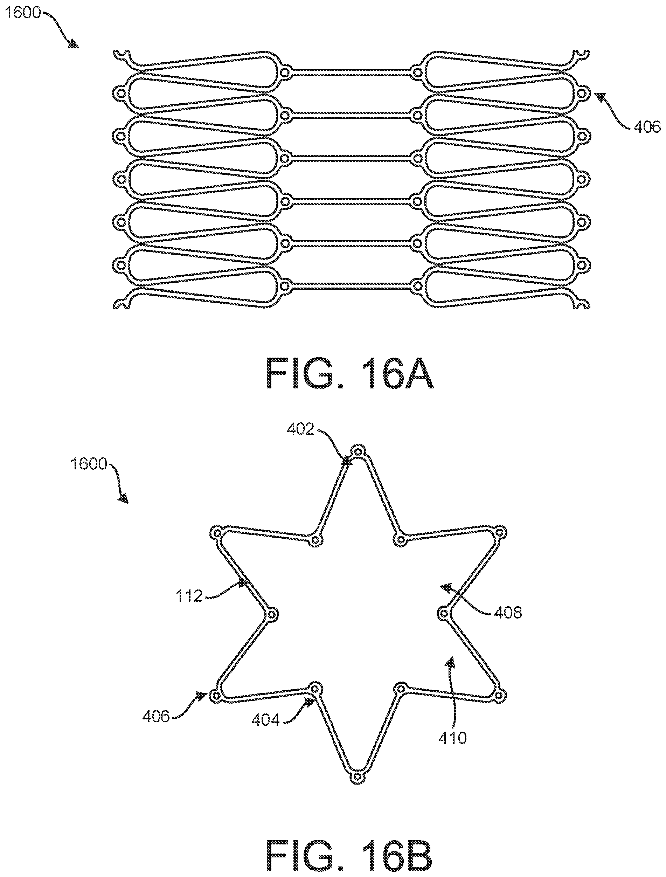

FIG. 16A shows a cut pattern of an example frame component 1600 that may be used in an implantable medical device for regulating blood pressure. FIG. 16B shows a side view of the frame component 1600, shown in FIG. 16A. The frame component 1600 may be used in place of one or both of the frame components 110, 120 shown and discussed above with reference to FIGS. 1-3. For example, the frame component 1600 may take place of the first and/or second frame components 110, 120 maintain a lumen through a conduit portion 130. The frame component 1600 may include elongate elements 112 that form the frame component 1600. In certain instances, the elongate elements 112 may be attached to one another via a membrane (not shown). In addition and as discussed in detail above, the membrane such as an expanded polytetrafluoroethylene (ePTFE) membrane, may connect frame components 1600 and form the conduit portion 130.

The membrane may span gaps between the elongate elements 112, and, in certain instances, may be arranged on at least a tissue engaging side of the frame component 1600. In these instances, the membrane is configured to lessens frame erosion potential of the frame component 1600. The elongate elements 112 may conform to the tissue surfaces surrounding the septum. In addition, the elongate elements 112 may lay flat against the tissue surfaces.

In certain instances, the elongate elements 112 form a star shape as is shown in FIG. 16B. The star-shaped frame component 1600 includes outer apices 402 and inner apices 404. In certain instances, the frame component 1600 may include one or more eyelets 406 arranged with at least one of the outer apices 402 and the inner apices 404. The frame component 1600 also includes an exterior side 408 and an interior side 410. In certain instances, the one or more eyelets 406 may be arranged on either side of the inner apices 404 and/or the outer apices 402. In certain instances, each of the inner apices 404 includes one of the eyelets 406 arranged on the exterior side 408 of the frame 1600. In addition, each of the outer apices 402 may include one of the eyelets 406 arranged on the interior side 410 of the frame 1600 in certain instances. In other instances, one of the eyelets 406 may be arranged on the exterior side 408 of the frame 1600 without eyelets arranged on the interior side 410 of the frame 1600. In yet other instances, one of the eyelets 406 may be arranged on the interior side 410 of the frame 1600 without eyelets 406 arranged on the exterior side 408 of the frame 1600. The eyelets 406 may be configured to interface with a delivery system to facilitate deployment of the frame component 1600.

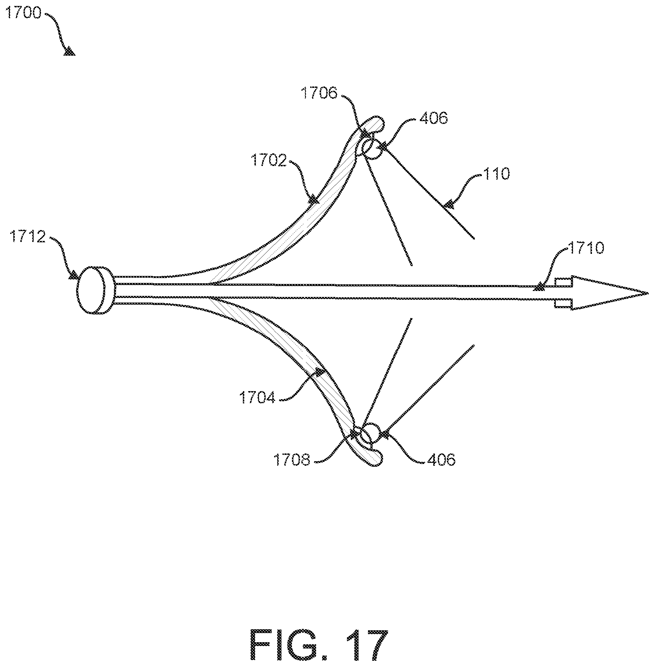

FIG. 17 shows an example deployment system 1700 and frame component 110, in accordance with the claimed delivery system. The deployment system 1700 may include engagement elements 1702, 1704 that are configured to engage with the frame component 110. As discussed in detail above, the frame component 110 may include eyelets 406. The engagement elements 1702, 1704, which may be formed of hypotubes or a cut-tube similar structure, may be configured to engage with one or more of the eyelets 406 of the frame component 110.

In certain instances, the delivery system 1700 may also include release lines 1706, 1708 that may be arranged within the engagement elements 1702, 1704. The release lines 1706, 1708 may be arranged through the eyelets 406 as shown. In certain instances, the release lines 1706, 1708 may be withdrawn to release the frame component 110 from the delivery system 1700. When engaged with the frame component 110, the engagement elements 1702, 1704 may be withdrawn to collapse the frame component 100 toward a catheter 1710. In certain instances, tension may be applied to the engagement elements 1702, 1704 (or elements coupled to the engagement elements 1702, 1704) that collapse the engagement elements 1702, 1704 and the frame component 110 toward the catheter 1710. In certain instances, the delivery system 1700 may include a delivery sheath 1712 that the engagement elements 1702, 1704 and the frame component 110 (and medical device which may include the frame component(s) 110) may be collapsed within. Withdrawal of the engagement elements 1702, 1704 within the delivery sheath 1712 may collapse the engagement elements 1702, 1704.

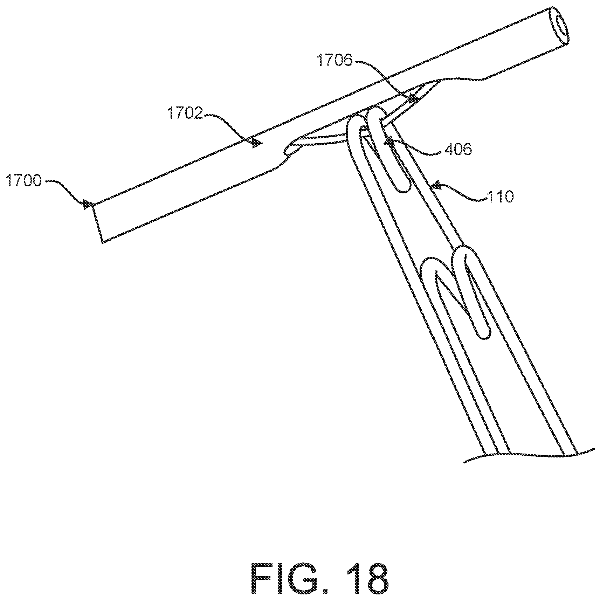

FIG. 18 shows an end portion of an example deployment system 1700 and frame component 110, in accordance with the claimed delivery system. An engagement element 1702 and release line 1706 are shown with the frame component 110. Th release line 1706 is arranged through the eyelet 406. As noted above, the release line 1706 may be withdrawn to uncouple the frame component 110 from the delivery system 1700 and the engagement element 1702.

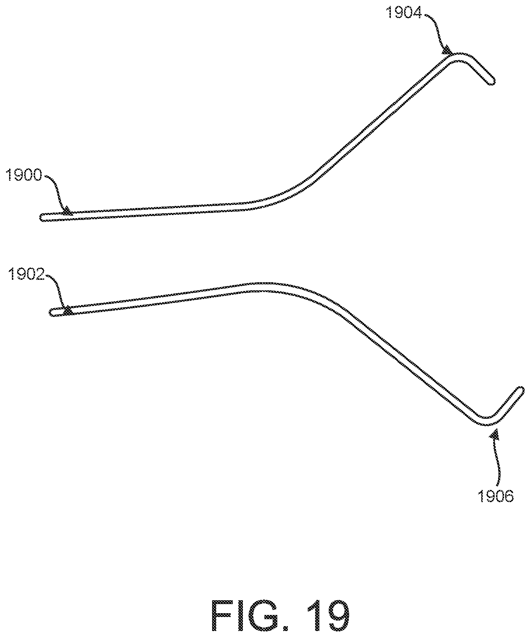

FIG. 19 shows example graspers 1900, 1902 for recapturing a frame component. The graspers 1900, 1902 may be used to grab or couple to a frame component or medical device. The graspers 1900, 1902 may be used to recapture the frame component or medical device or facilitate deployment of the frame component or medical device. The graspers 1900, 1902 may include hook ends 1904, 1906 that may be arranged through eyelets of a frame component.

## Claims

1. An implantable medical device (100), comprising:
a first frame component (110) configured to conform to an anatomy of a patient;
a second frame (120) component configured to conform to an anatomy of a patient wherein the first frame component (110) and the second frame (120), and at least one of the first frame component (110) and the second frame component (120) includes inner apices (404) and outer apices (402); and
wherein at least one of the first frame component and the second frame component includes a star shape (400) having the inner apices (404) and the outer apices (402) and the at least one of the first frame component (110) and the second frame component (120) includes an exterior side (408) and an interior side (410), and one or more eyelets (406) are arranged on either side of at least one of the inner apices (404) and the outer apices (402);
wherein the one or more eyelets (406) arranged with the outer apices (402) are open to the interior side (410) of the at least one of the first frame component (110) and the second frame component (120); and
wherein the implantable medical device further comprises a conduit portion (130) arranged between the first frame component (110) and the second frame component (120), the conduit portion (130) including a membrane (132) connecting the first frame component (110) and the second frame component (120).

2. The device of claim 1, wherein the one or more eyelets (406) arranged with the inner apices (404) are open to the exterior side (408) of the at least one of the first frame component (110) and the second frame component (120).

3. The device of claim 1, wherein the star shape is a curved star shape.

4. The device of any one of claims 1-3, wherein the eyelets are oval shaped.

5. The device of claim 1, wherein at least one of the first frame component (110) and the second frame component (120) includes the star shape having elongate elements (112) with variable widths or one or more curvatures between the outer apices (402) and the inner apices (404).

6. A delivery system for deploying an implantable medical device according to claims 1 to 5 for regulating blood pressure between a left and right atrium of a heart, the system comprising:
an implantable medical device according to claims 1 to 5;
one or more engagement elements (1702, 1704); and
one or more release lines (1706, 1708) arranged through the engagement elements (1702, 1704) and configured to pass through the one or more eyelets (406) to couple the at least one of the first frame component (110) and the second frame (120) component to the delivery system.

7. The system of claim 6, wherein the one or more engagement elements (1702, 1704) are configured to collapse the at least one of the first frame component (110) and the second frame component (120) toward a catheter (1710).

8. The system of any one of claims 6 to 7, wherein the release lines (1706, 1708) are configured to withdraw from the one or more eyelets (406) to release the frame component (110, 120) from the delivery system.

## Patentansprüche

1. Implantierbare medizinische Vorrichtung (100), umfassend:
eine erste Rahmenkomponente (110), die dazu konfiguriert ist, sich an eine Anatomie eines Patienten anzupassen;
eine Komponente eines zweiten Rahmens (120), die dazu konfiguriert ist, sich an eine Anatomie eines Patienten anzupassen, wobei die erste Rahmenkomponente (110) und der zweite Rahmen (120) und mindestens eine von der ersten Rahmenkomponente (110) und der zweiten Rahmenkomponente (120) innere Scheitelpunkte (404) und äußere Scheitelpunkte (402) beinhalten; und
wobei mindestens eine von der ersten Rahmenkomponente und der zweiten Rahmenkomponente eine Sternform (400) mit den inneren Scheitelpunkten (404) und den äußeren Scheitelpunkten (402) beinhaltet und die mindestens eine von der ersten Rahmenkomponente (110) und der zweiten Rahmenkomponente (120) eine Außenseite (408) und eine Innenseite (410) beinhaltet und eine oder mehrere Ösen (406) auf jeder Seite von mindestens einem von den inneren Scheitelpunkten (404) und den äußeren Scheitelpunkten (402) angeordnet sind;
wobei die eine oder mehreren Ösen (406), die mit den äußeren Scheitelpunkten (402) angeordnet sind, zu der Innenseite (410) der mindestens einen von der ersten Rahmenkomponente (110) und der zweiten Rahmenkomponente (120) offen sind; und
wobei die implantierbare medizinische Vorrichtung ferner einen Leitungsabschnitt (130) umfasst, der zwischen der ersten Rahmenkomponente (110) und der zweiten Rahmenkomponente (120) angeordnet ist, wobei der Leitungsabschnitt (130) eine Membran (132) beinhaltet, die die erste Rahmenkomponente (110) und die zweite Rahmenkomponente (120) verbindet.

2. Vorrichtung nach Anspruch 1, wobei die eine oder mehreren Ösen (406), die mit den inneren Scheitelpunkten (404) angeordnet sind, zu der Außenseite (408) der mindestens einen von der ersten Rahmenkomponente (110) und der zweiten Rahmenkomponente (120) offen sind.

3. Vorrichtung nach Anspruch 1, wobei die Sternform eine gekrümmte Sternform ist.

4. Vorrichtung nach einem der Ansprüche 1-3, wobei die Ösen ovalförmig sind.

5. Vorrichtung nach Anspruch 1, wobei mindestens eine von der ersten Rahmenkomponente (110) und der zweiten Rahmenkomponente (120) die Sternform mit länglichen Elementen (112) mit variablen Breiten oder einer oder mehreren Krümmungen zwischen den äußeren Scheitelpunkten (402) und den inneren Scheitelpunkten (404) beinhaltet.

6. Abgabesystem zum Entfalten einer implantierbaren medizinischen Vorrichtung nach Anspruch 1 bis 5 zum Regulieren von Blutdruck zwischen einem linken und rechten Vorhof eines Herzens, wobei das System Folgendes umfasst:
eine implantierbare medizinische Vorrichtung nach Anspruch 1 bis 5;
ein oder mehrere Eingriffselemente (1702, 1704); und
eine oder mehrere Freigabeschnüre (1706, 1708), die durch die Eingriffselemente (1702, 1704) angeordnet sind und dazu konfiguriert sind, durch die eine oder mehreren Ösen (406) zu verlaufen, um die mindestens eine von der ersten Rahmenkomponente (110) und der Komponente des zweiten Rahmens (120) an das Abgabesystem zu koppeln.

7. System nach Anspruch 6, wobei das eine oder die mehreren Eingriffselemente (1702, 1704) dazu konfiguriert sind, die mindestens eine von der ersten Rahmenkomponente (110) und der zweiten Rahmenkomponente (120) zu einem Katheter (1710) hin zusammenzufalten.

8. System nach einem der Ansprüche 6 bis 7, wobei die Freigabeschnüre (1706, 1708) dazu konfiguriert sind, sich von der einen oder den mehreren Ösen (406) zurückziehen, um die Rahmenkomponente (110, 120) von dem Abgabesystem zu lösen.

## Revendications

1. Dispositif médical implantable (100), comprenant :
un premier composant de cadre (110) conçu pour se conformer à l'anatomie d'un patient ;
un second composant de cadre (120) conçu pour se conformer à l'anatomie d'un patient, ledit premier composant de cadre (110) et ledit second cadre (120), et au moins un composant de cadre parmi le premier composant de cadre (110) et le second composant de cadre (120) comprenant des sommets internes (404) et des sommets externes (402) ; et
au moins un composant de cadre parmi le premier composant de cadre et le second composant de cadre comprenant une forme d'étoile (400) comportant les sommets internes (404) et les sommets externes (402) et ledit au moins un composant de cadre parmi le premier composant de cadre (110) et le second composant de cadre (120) comprenant un côté extérieur (408) et un côté intérieur (410), et un ou plusieurs œillets (406) étant agencés de chaque côté d'au moins un sommet parmi les sommets internes (404) et les sommets externes (402) ;
lesdits un ou plusieurs œillets (406) agencés avec les sommets externes (402) étant ouverts sur le côté intérieur (410) dudit au moins un composant de cadre parmi le premier composant de cadre (110) et le second composant de cadre (120) ; et
ledit dispositif médical implantable comprenant en outre une partie de conduit (130) disposée entre le premier composant de cadre (110) et le second composant de cadre (120), ladite partie de conduit (130) comprenant une membrane (132) reliant le premier composant de cadre (110) et le second composant de cadre (120).

2. Dispositif de la revendication 1, lesdits un ou plusieurs œillets (406) agencés avec les sommets internes (404) étant ouverts sur le côté extérieur (408) dudit au moins un composant de cadre parmi le premier composant de cadre (110) et le second composant de cadre (120).

3. Dispositif de la revendication 1, ladite forme d'étoile étant une forme d'étoile incurvée.

4. Dispositif de l'une quelconque des revendications 1 à 3, lesdits œillets étant de forme ovale.

5. Dispositif de la revendication 1, au moins un composant de cadre parmi le premier composant de cadre (110) et le second composant de cadre (120) comprenant la forme d'étoile comportant des éléments allongés (112) avec des largeurs variables ou une ou plusieurs courbures entre les sommets externes (402) et les sommets internes (404).

6. Système d'administration permettant de déployer un dispositif médical implantable selon les revendications 1 à 5 afin de réguler la pression artérielle entre l'oreillette gauche et l'oreillette droite d'un cœur, le système comprenant :
un dispositif médical implantable selon les revendications 1 à 5 ;
un ou plusieurs éléments de mise en prise (1702, 1704) ; et
une ou plusieurs lignes de libération (1706, 1708) agencées à travers les éléments de mise en prise (1702, 1704) et conçues pour passer à travers lesdits un ou plusieurs œillets (406) de manière à coupler ledit au moins un composant de cadre parmi le premier composant de cadre (110) et le second composant de cadre (120) au système d'administration.

7. Système de la revendication 6, lesdits un ou plusieurs éléments de mise en prise (1702, 1704) étant conçus pour replier ledit au moins un composant de cadre parmi le premier composant de cadre (110) et le second composant de cadre (120) en direction d'un cathéter (1710).

8. Système de l'une quelconque des revendications 6 à 7, lesdites lignes de libération (1706, 1708) étant conçues pour se retirer desdits un ou plusieurs œillets (406) de manière à libérer le composant de cadre (110, 120) du système d'administration.
